(19) Europäisches Patentamt
European Patent Office
Office européen des brevets

(11) Veröffentlichungsnummer: **0 029 002**
**B1**

(12) # EUROPÄISCHE PATENTSCHRIFT

(45) Veröffentlichungstag der Patentschrift: **23.03.83**

(51) Int. Cl.³: **C 07 C 117/08**, A 01 N 33/26, C 07 C 121/34, C 07 C 121/38

(21) Anmeldenummer: **80810313.9**

(22) Anmeldetag: **13.10.80**

(54) **Phenylacetate, Verfahren zu ihrer Herstellung und ihre Verwendung in der Schädlingsbekämpfung.**

(30) Priorität: **17.10.79 CH 9335/79**
**04.06.80 CH 4332/80**

(43) Veröffentlichungstag der Anmeldung: **20.05.81 Patentblatt 81/20**

(45) Bekanntmachung des Hinweises auf die Patenterteilung: **23.03.83 Patentblatt 83/12**

(84) Benannte Vertragsstaaten: **AT BE CH DE FR GB IT LI NL**

(56) Entgegenhaltungen: **keine**

(73) Patentinhaber: **CIBA-GEIGY AG, Patentabteilung Postfach, CH-4002 Basel (CH)**

(72) Erfinder: **Fischer, Hanspeter, Dr., Burggartenstrasse 14, CH-4103 Bottmingen (CH)**
Erfinder: **Thummel, Rudolph C., Route d'Alle 424, CH-2892 Courgenay (CH)**
Erfinder: **Wehrli, Rudolf, Dr., Dianastrasse 2, CH-4310 Rheinfelden (CH)**

EP 0 029 002 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Phenylacetate, Verfahren zu ihrer Herstellung und ihre Verwendung in der Schädlingsbekämpfung

Die vorliegende Erfindung betrifft α-Phenylacetate, Verfahren zu ihrer Herstellung und ihre Verwendung in der Schädlingsbekämpfung.

Die Phenylacetate haben die Formel

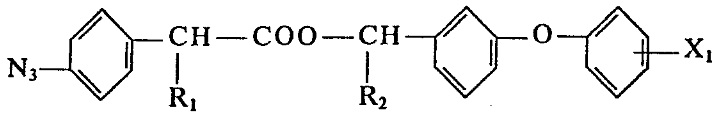

(I)

worin

$R_1$ Isopropyl oder Cyclopropyl,
$R_2$ Wasserstoff, Cyano, Äthinyl, Prop-1-inyl oder $-CSNH_2$ und
$X_1$ Wasserstoff, Methyl, Fluor oder Chlor

bedeuten.

Wegen ihrer Wirkung von besonderer Bedeutung sind Verbindungen der Formel I, worin

$R_1$ Isopropyl oder Cyclopropyl,
$R_2$ Wasserstoff, Cyano, Äthinyl, Prop-1-inyl oder $-CSNH_2$ und
$X_1$ Wasserstoff, p-Fluor oder p-Chlor

bedeuten.

Insbesondere von Bedeutung sind aber Verbindungen der Formel I, worin

$R_1$ Isopropyl,
$R_2$ Cyano und
$X_1$ Wasserstoff, p-Fluor oder p-Chlor

bedeuten.

Die Verbindungen der Formel I werden nach an sich bekannten Methoden z. B. wie folgt hergestellt:

1) 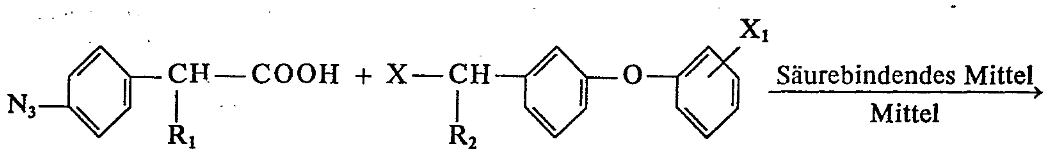

I

(II) (III)

2) 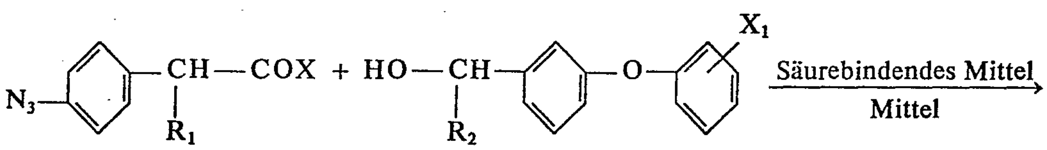

I

(IV) (V)

3) 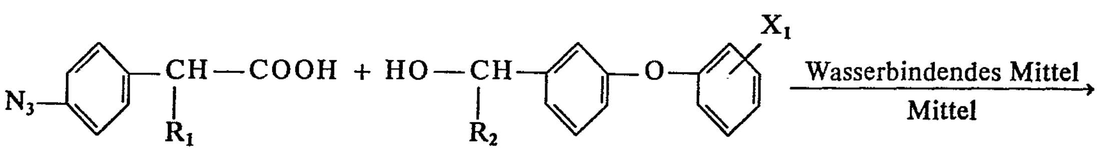

I

(II) (V)

4) 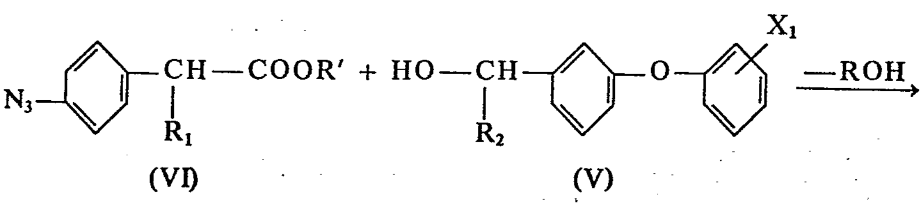

I

(VI) (V)

In den Formeln II bis VI haben $R_1$, $R_2$ und $X_1$ die für die Formel I angegebene Bedeutung.

In den Formeln III und IV steht X für ein Halogenatom, insbesondere Chlor oder Brom, und in der Formel VI steht R' für $C_1-C_4$-Alkyl, insbesondere für Methyl oder Äthyl. Als säurebindendes Mittel für die Verfahren 1 und 2 kommen insbesondere tertiäre Amine, wie Trialkylamin und Pyridin, ferner Hydroxide, Oxide, Carbonate und Bicarbonate von Alkali- und Erdalkalimetallen sowie Alkalimetallalkoholate, wie z. B. Kalium-t.butylat und Natriummethylat in Betracht. Als wasserbindendes Mittel für das Verfahren 3 kann z. B. Dicyclohexylcarbodiimid verwendet werden. Die Verfahren 1 bis 4 werden bei einer Reaktionstemperatur zwischen −10 und 120°C, meist zwischen 20 und 80°C bei normalem oder erhöhtem Druck und vorzugsweise in einem inerten Lösungs- oder Verdünnungsmittel durchgeführt. Als Lösungs- oder Verdünnungsmittel eignen sich z. B. Äther und ätherartige Verbindungen, wie Diäthyläther, Dipropyläther, Dioxan, Dimethoxyäthan und Tetrahydrofuran; Amide, wie N,N-dialkylierte Carbonsäureamide; aliphatische, aromatische sowie halogenierte Kohlenwasserstoffe, insbesondere Benzol, Toluol, Xylole, Chloroform und Chlorbenzol; Nitrile wie Acetonitril; Dimethylsulfoxid und Ketone wie Aceton und Methyläthylketon.

Die Ausgangsstoffe der Formeln III und V sind bekannt, während die Ausgangsstoffe der Formeln II, IV und VI neu sind. Alle Ausgangsstoffe können aber nach bekannten Methoden hergestellt werden.

Die Verbindungen der Formel I liegen als Gemisch von verschiedenen optisch aktiven Isomeren vor, wenn bei der Herstellung nicht einheitlich optisch aktive Ausgangsmaterialien verwendet wurden. Die verschiedenen Isomerengemische können nach bekannten Methoden in die einzelnen Isomeren aufgetrennt werden. Unter der Verbindung der Formel I versteht man sowohl die einzelnen Isomeren, als auch deren Gemische.

Die Verbindungen der Formel I eignen sich zur Bekämpfung von verschiedenartigen tierischen und pflanzlichen Schädlingen.

Insbesondere eignen sich die Verbindungen der Formel I zur Bekämpfung von Insekten, phytopathogenen Milben und von Zecken z. B. der Ordnungen Lepidoptera, Coleoptera, Homoptera, Heteroptera, Diptera, Acarina, Thysanoptera, Orthoptera, Anoplura, Siphonaptera, Mallophaga, Thysanura, Isoptera, Psocoptera und Hymenoptera.

Vor allem eignen sich Verbindungen der Formel I zur Bekämpfung von pflanzenschädigenden Insekten, insbesondere pflanzenschädigenden Fraßinsekten, in Zier- und Nutzpflanzen, insbesondere in Baumwollkulturen (z. B. gegen Spodoptera littoralis und Heliothis virescens) und Gemüsekulturen (z. B. gegen Leptinotarsa decemlineata und Myzus persicae).

Wirkstoffe der Formel I zeigen auch eine sehr günstige Wirkung gegen Fliegen, wie z. B. Musca domestica und Mückenlarven.

Die akarizide bzw. insektizide Wirkung läßt sich durch Zusatz von anderen Insektiziden und/oder Akariziden wesentlich verbreitern und an gegebene Umstände anpassen. Als Zusätze eignen sich z. B. org. Phosphorverbindungen; Nitrophenole und deren Derivate; Formamidine; Harnstoffe; andere pyrethrinartige Verbindungen sowie Karbamate und chlorierte Kohlenwasserstoffe.

Mit besonderem Vorteil werden Verbindungen der Formel I auch mit Substanzen kombiniert, welche einen synergistischen oder verstärkenden Effekt auf Pyrethroide ausüben. Beispiele solcher Verbindungen sind u. a. Piperonylbutoxid, Propinyläther, Propinyloxime, Propinylcarbamate und Propinylphosphonate, 2-(3,4-Methylendioxyphenoxy)-3,6,9-trioxaundecan (Sesamex resp. Sesoxane), S,S,S-Tributylphosphorotrithioate, 1,2-Methylendioxy-4-(2-(octylsulfinyl)-propyl)-benzol.

Verbindungen der Formel I können für sich allein oder zusammen mit geeigneten Trägern und/oder Zuschlagstoffen eingesetzt werden. Geeignete Träger- und Zuschlagstoffe können fest oder flüssig sein und entsprechen den in der Formulierungstechnik üblichen Stoffen, wie z. B. natürlichen oder regenerierten Stoffen, Lösungs-, Dispergier-, Netz-, Haft-, Verdickungs-, Binde- und/oder Düngemitteln.

Die Herstellung erfindungsgemäßer Mittel erfolgt in an sich bekannter Weise durch inniges Vermischen und/oder Vermahlen der Wirkstoffe der Formel I mit den geeigneten Trägerstoffen, gebenenfalls unter Zusatz von gegenüber den Wirkstoffen inerten Dispergier- oder Lösungsmitteln. Die Wirkstoffe können in den folgenden Aufarbeitungsformen vorliegen und angewendet werden:

Feste Aufarbeitungsformen:

Stäubemittel, Streumittel, Granulate (Umhüllungsgranulate, Imprägnierungsgranulate und Homogranulate);

Flüssige Aufarbeitungsformen:

a) in Wasser dispergierbare Wirkstoffkonzentrate: Spritzpulver (wettable powders), Pasten, Emulsionen;
b) Lösungen.

Der Gehalt an Wirkstoff in den oben beschriebenen Mitteln liegt zwischen 0,1 bis 95%, dabei ist zu erwähnen, daß bei der Applikation aus dem Flugzeug oder mittels anderer geeigneter Applikationsgeräte Konzentrationen bis zu 99,5% oder sogar reiner Wirkstoff eingesetzt werden können. Die Wirkstoffe der Formel I können beispielsweise wie folgt formuliert werden (Teile bedeuten Gewichtsteile):

## Stäubemittel

Zur Herstellung eines a) 5%igen und b) 2%igen Stäubemittels werden die folgenden Stoffe verwendet:

| | | |
|---|---|---|
| a) | 5 Teile | Wirkstoff |
| | 95 Teile | Talkum; |
| b) | 2 Teile | Wirkstoff |
| | 1 Teil | hochdisperse Kieselsäure |
| | 97 Teile | Talkum. |

Der Wirkstoff wird mit den Trägerstoffen vermischt und vermahlen.

## Granulat

Zur Herstellung eines 5%igen Granulates werden die folgenden Stoffe verwendet:

| | |
|---|---|
| 5 Teile | Wirkstoff |
| 0,25 Teile | epoxydiertes Pflanzenöl |
| 0,25 Teile | Cetylpolyglykoläther |
| 3,50 Teile | Polyäthylenglykol |
| 91 Teile | Kaolin (Korngröße 0,3–0,8 mm). |

Die Aktivsubstanz wird mit epoxydiertem Pflanzenöl vermischt und mit 6 Teilen Aceton gelöst, hierauf wird Polyäthylenglykol und Cetylpolyglykoläther zugesetzt. Die so erhaltene Lösung wird auf Kaolin aufgesprüht und anschließend das Aceton im Vakuum eingedampft.

## Spritzpulver

Zur Herstellung eines a) 40%igen, b) und c) 25%igen, d) 10%igen Spritzpulvers werden folgende Bestandteile verwendet:

| | | |
|---|---|---|
| a) | 40 Teile | Wirkstoff |
| | 5 Teile | Ligninsulfonsäure-Natriumsalz |
| | 1 Teil | Dibutylnaphthalinsulfonsäure-Natriumsalz |
| | 54 Teile | Kieselsäure; |
| b) | 25 Teile | Wirkstoff |
| | 4,5 Teile | Calcium-Ligninsulfonat |
| | 1,9 Teile | Champagne-Kreide/Hydroxyäthylcellulose-Gemisch (1 : 1) |
| | 1,5 Teile | Natrium-dibutyl-naphthalinsulfonat |
| | 19,5 Teile | Kieselsäure |
| | 19,5 Teile | Champagne-Kreide |
| | 28,1 Teile | Kaolin; |
| c) | 25 Teile | Wirkstoff |
| | 2,5 Teile | Isooctylphenoxy-polyäthylen-äthanol |
| | 1,7 Teile | Champagne-Kreide/Hydroxyäthylcellulose-Gemisch (1 : 1) |
| | 8,3 Teile | Natriumaluminiumsilikat |
| | 16,5 Teile | Kieselgur |
| | 46 Teile | Kaolin; |
| d) | 10 Teile | Wirkstoff |
| | 3 Teile | Gemisch der Natriumsalze von gesättigten Fettalkoholsulfaten |
| | 5 Teile | Naphthalinsulfonsäure/Formaldehyd-Kondensat |
| | 82 Teile | Kaolin. |

Der Wirkstoff wird in geeigneten Mischern mit dem Zuschlagstoff innig vermischt und auf entsprechenden Mühlen und Walzen vermahlen. Man erhält Spritzpulver, die sich mit Wasser zu Suspensionen jeder gewünschten Konzentration verdünnen lassen.

## Emulgierbare Konzentrate

Zur Herstellung eines a) 10%igen, b) 25%igen und c) 50%igen emulgierbaren Konzentrates werden folgende Stoffe verwendet:

| | | |
|---|---|---|
| a) | 10 Teile | Wirkstoff |
| | 3,4 Teile | epoxydiertes Pflanzenöl |
| | 3,4 Teile | eines Kombinationsemulgators, bestehend aus Fettalkoholpolyglykoläther und Alkylarylsulfonat-Calcium-Salz |
| | 40 Teile | Dimethylformamid |
| | 43,2 Teile | Xylol; |
| b) | 25 Teile | Wirkstoff |
| | 2,5 Teile | epoxydiertes Pflanzenöl |
| | 10 Teile | eines Alkylarylsulfonat/Fettalkoholpolyglykoläther-Gemisches |
| | 5 Teile | Dimethylformamid |
| | 57,5 Teile | Xylol; |
| c) | 50 Teile | Wirkstoff |
| | 4,2 Teile | Tributylphenol-Polyglykoläther |
| | 5,8 Teile | Calcium-Dodecylbenzolsulfonat |
| | 20 Teile | Cyclohexanon |
| | 20 Teile | Xylol. |

Aus solchen Konzentrationen können durch Verdünnen mit Wasser Emulsionen jeder gewünschten Konzentration hergestellt werden.

## Sprühmittel

Zur Herstellung eines a) 5%igen und b) 95%igen Sprühmittels werden die folgenden Bestandteile verwendet:

| | | |
|---|---|---|
| a) | 5 Teile | Wirkstoff |
| | 1 Teil | epoxydiertes Pflanzenöl |
| | 94 Teile | Benzin (Siedegrenzen 160–190°C); |
| b) | 95 Teile | Wirkstoff |
| | 5 Teile | epoxydiertes Pflanzenöl. |

## Beispiel 1

### Herstellung von (–)-2-(4-Azidophenyl)-3-methylbuttersäure-(±)-α-cyano-3-phenoxybenzylester

#### a) (–)-2-(4-Azidophenyl)-3-methylbuttersäure

Eine Lösung von 65,7 g (±)-2-(4-Azidophenyl)-3-methylbuttersäure in 800 ml 63%igem Äthanol wird auf 60°C erwärmt und unter Rühren zu einer Lösung von 36,3 g (+)-Phenyläthylamin in 560 ml 63%igem Äthanol bei 60°C gegeben. Die erhaltene Lösung wird 12 Stunden auf Raumtemperatur abgekühlt, das auskristallisierte Salz (43 g) abfiltriert und 2mal aus 63%igem Äthanol umkristallisiert. Man erhält 12,5 g Salz, aus welchem die gewünschte Säure durch Behandeln mit verd. Salzsäure, Extraktion mit Essigester, Trocknen der organischen Phase über $Na_2SO_4$ und Eindampfen, isoliert werden kann. Nach zweimaligem Umkristallisieren aus Hexan erhält man die reine (–)-2-(4-Azidophenyl)-3-methylbuttersäure mit einem Schmelzpunkt von 95–97°C und einer spezifischen Drehung $[\alpha]_D^{20°}$ von –48° ($CHCl_3$, c = 0,75).

Durch weitere Umkristallisation des Salzes veränderte sich der Drehwert der daraus gewonnenen Säure nicht.

#### b) (–)-2-(4-Azidophenyl)-3-methylbuttersäurechlorid

1,5 g der nach a) erhaltenen Säure und 1,3 g Oxalylchlorid werden in 35 ml Hexan auf 60°C erwärmt, mit 1 Tropfen DMF versetzt und 1 Stunde bei 60°C gerührt. Die kalte Lösung wird abdekantiert und aus

Rotationsverdampfer eingedampft. Das so erhaltene (−)-2-(4-Azidophenyl)-3-methylbuttersäurechlorid wird roh weiterverarbeitet.

c) (−)-2-(4-Azidophenyl)-3-methylbuttersäure-(±)-α-cyano-3-phenoxybenzylester

1,6 g des nach b) erhaltenen Säurechlorids und 1,5 g α-Cyano-3-phenoxybenzylalkohol werden in 20 ml Toluol abs. bei Raumtemperatur mit 0,6 g Pyridin in 10 ml Toluol abs. versetzt und über Nacht bei Raumtemperatur gerührt. Die Reaktionsmischung wird mit Äther verdünnt, mit Natriumbicarbonatlösung, 2N Salzsäure und Sole gewaschen, über $Na_2SO_4$ getrocknet und eingedampft. Nach Chromatographie an Kieselgel mit Toluol/Essigester (10 : 1) erhält man die reine Verbindung der Formel

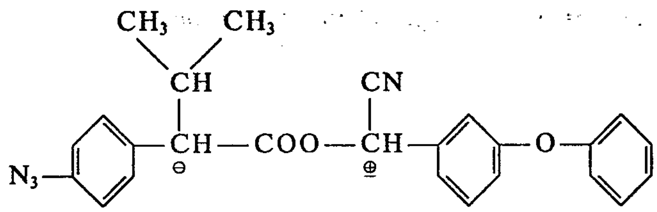

mit einem Brechungsindex von $n_D^{20°} = 1,5775$, einer opt. Drehung $[\alpha]_D^{20°} = +17°$ ($CHCl_3$, c=0,72) und einem NMR (60 MHz, $CDCl_3$):

| | |
|---|---|
| 0,55−1,25 ppm | (m; 6 H, 2 $CH_3$) |
| 1,8−2,7 ppm | (m; 1 H) |
| 3,25 ppm | (d; J = 10 Hz; 1 H) |
| 6,3−6,4 ppm | (2 S; 1H) |
| 6,7−7,6 ppm | (m; 13 H) |

Beispiel 2

Herstellung von (+)-2-(4-Azidophenyl)-3-methylbuttersäure-(±)-α-cyano-3-phenoxybenzylester

a) (+)-2-(4-Azidophenyl)-3-methylbuttersäure

Die erste Mutterlauge der Racematspaltung in Beispiel 1a) wird eingedampft, die Säure mit verd. Salzsäure freigesetzt, mit Essigester extrahiert, der organ. Extrakt über $Na_2SO_4$ getrocknet und eingedampft. 28,3 g dieser Säure werden bei 60°C in 330 ml 63%igem Alkohol gelöst und unter Rühren zu einer Lösung von 15,6 g (−)-Phenyläthylamin in 255 ml 63%igem Äthanol bei 60°C gegeben. Man läßt über Nacht bei 30°C auskristallisieren, filtriert das ausgeschiedene Salz (23 g) ab und kristallisiert dieses einmal aus 63%igem Alkohol um. Man erhält 14 g Salz, aus welchem die gewünschte (+)-2-(4-Azidophenyl)-3-methylbuttersäure auf oben beschriebenem Weg freigesetzt und durch zweimaliges Umkristallisieren rein erhalten wird (Smp. 95−97°C; $[\alpha]_D^{20°} = +48°$ [$CHCl_3$, c=1,30]).

b) (−)-2-(4-Azidophenyl)-3-methylbuttersäurechlorid

2 g der nach a) erhaltenen Säure und 1,7 g Oxalylchlorid werden in 35 ml Hexan auf 60°C erwärmt, mit 1 Tropfen DMF versetzt und 1 Stunde bei 60°C gerührt. Die kalte Lösung wurde abdekantiert und am Rotationsverdampfer eingedampft. Das so erhaltene (+)-2-(4-Azidophenyl)-3-methylbuttersäurechlorid wird roh weiterverarbeitet.

c) (+)-2-(4-Azidophenyl)-3-methylbuttersäure-(±)-α-cyano-3-phenoxybenzylester

2,1 g des nach b) erhaltenen Säurechlorids und 1,98 g α-Cyano-3-phenoxybenzylalkohol werden in 20 ml Toluol abs. bei Raumtemperatur mit 0,8 g Pyridin in 10 ml Toluol abs. versetzt und über Nacht bei Raumtemperatur gerührt. Die Reaktionsmischung wird mit Äther verdünnt, mit Natriumbicarbonatlösung, 2N Salzsäure und Sole gewaschen, über $Na_2SO_4$ getrocknet und eingedampft. Nach Chromatographie an Kieselgel mit Toluol/Essigester (10 : 1) erhält man die reine Verbindung der Formel

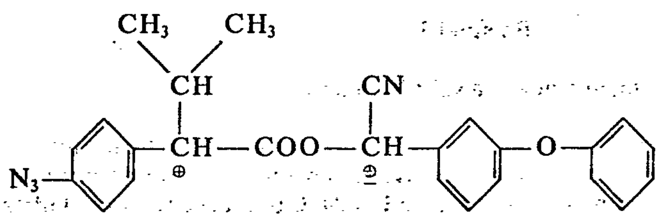

mit einem Brechungsindex von $n_D^{22°} = 1,5785$, einer opt. Drehung $[\alpha]_D^{20°} = -17°$ ($CHCl_3$, c=0,64) und einem NMR (60 MHz, $CDCl_3$):

- 0,5–1,2 ppm (m; 6 H, 2 $CH_3$)
- 1,85–2,65 ppm (m; 1 H)
- 3,25 ppm (d; J = 10 Hz; 1 H)
- 6,3–6,4 ppm (25; 1 H)
- 6,65–7,7 ppm (m; 13 H)

Auf analoge Weise werden auch folgende Verbindungen hergestellt:

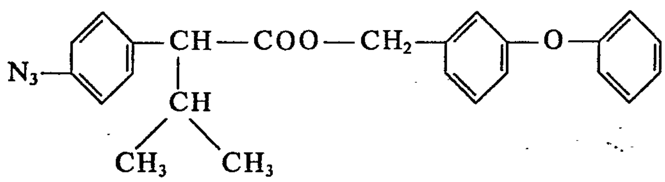

$n_D^{24,5°}$: 1,5772

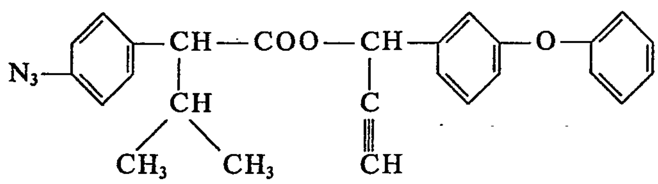

$n_D^{25°}$: 1,5826

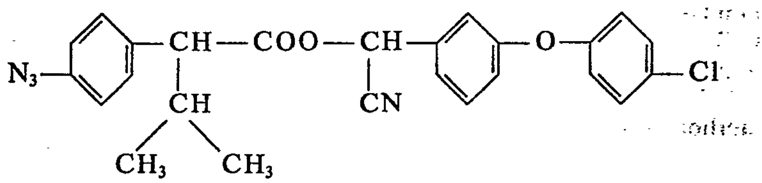

$n_D^{25°}$: 1,5820

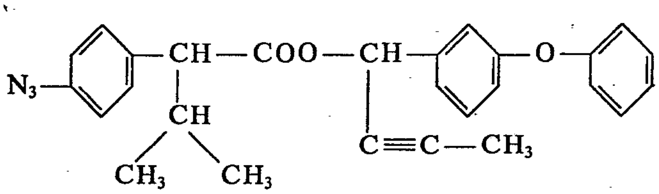

$n_D^{24,5°}$: 1,5827

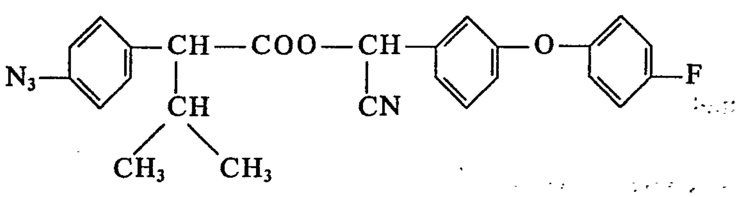

$n_D^{24,5°}$: 1,5680

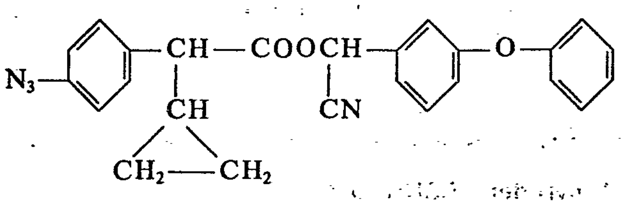

$n_D^{22,5°}$: 1,5665

### Beispiel 3

### Insektizide Fraßgift-Wirkung

Baumwollpflanzen wurden mit einer 0,05%igen wäßrigen Wirkstoffemulsion besprüht.

Nach dem Antrocknen des Belages wurden die Baumwollpflanzen je mit Spodoptera littoralis- und Helothis virescens-Larven $L_3$ besetzt. Der Versuch wurde bei 24°C und 60% relativer Luftfeuchtigkeit durchgeführt.

Die Verbindungen gemäß Beispiel 1 und 2 zeigten im obigen Test eine gute insektizide Fraßgift-Wirkung gegen Spodoptera- und Heliothis-Larven.

### Beispiel 4

### Akarizide Wirkung

Phaseolus vulgaris Pflanzen wurden 12 Stunden vor dem Test auf akarizide Wirkung mit einem infestierten Blattstück aus einer Massenzucht von Tetranychus urticae belegt. Die übergelaufenen beweglichen Stadien wurden aus einem Chromatographiezerstäuber mit den emulgierten Testpräparaten derart besprüht, daß kein Ablaufen der Spritzbrühe eintrat. Nach 2 und 7 Tagen wurden Larven, Adulte und Eier unter dem Binokular auf lebende und tote Individuen ausgewertet und das Ergebnis in Prozenten ausgedrückt. Während der »Haltezeit« standen die behandelten Pflanzen in Gewächshauskabinen bei 25°C.

Die Verbindungen gemäß Beispiel 1 und 2 wirkten im obigen Test gegen Adulte, Larven und Eier von Tetranychus urticae.

### Beispiel 5

### a) Rhipicephalus bursa

Je 5 adulte Zecken bzw. 50 Zeckenlarven wurden in ein Glasröhrchen gezählt und für 1 bis 2 Minuten in 2 ml einer wäßrigen Emulsion aus einer Verdünnungsreihe mit je 100, 10, 1 oder 0,1 ppm Testsubstanz getaucht. Das Röhrchen wurde dann mit einem genormten Wattebausch verschlossen und auf den Kopf gestellt, damit die Wirkstoffemulsion von der Watte aufgenommen werden konnte.

Die Auswertung erfolgte bei den Adulten nach 2 Wochen und bei den Larven nach 2 Tagen. Für jeden Versuch liefen 2 Wiederholungen.

### b) Boophilus microplus (Larven)

Mit einer analogen Verdünnungsreihe wie beim Test a) wurden mit je 20 sensiblen resp. OP-resistenten Larven Versuche durchgeführt. (Die Resistenz bezieht sich auf die Verträglichkeit von Diazinon.) Die Verbindungen gemäß Beispiel 1 und 2 wirkten in diesem Test gegen Adulte und Larven von Rhipicephalus bursa und sensible resp. OP-resistente Larven von Boophilus microplus.

**Patentansprüche**

1. Eine Verbindung der Formel

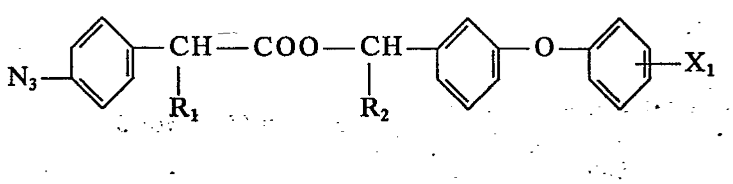

worin

$R_1$ Isopropyl oder Cyclopropyl,
$R_2$ Wasserstoff, Cyano, Äthinyl, Prop-1-inyl oder $-CSNH_2$ und
$X_1$ Wasserstoff, Methyl, Fluor oder Chlor

bedeuten.

2. Eine Verbindung gemäß Anspruch 1, worin

$R_1$ Isopropyl oder Cyclopropyl,
$R_2$ Wasserstoff, Cyano, Äthinyl, Prop-1-inyl oder $-CSNH_2$ und
$X_1$ Wasserstoff, p-Fluor oder p-Chlor

bedeuten.

3. Eine Verbindung gemäß Anspruch 2, worin

$R_1$ Isopropyl,
$R_2$ Cyano und
$X_1$ Wasserstoff, p-Fluor oder p-Chlor

bedeuten.

4. Die Verbindung gemäß Anspruch 3 der Formel

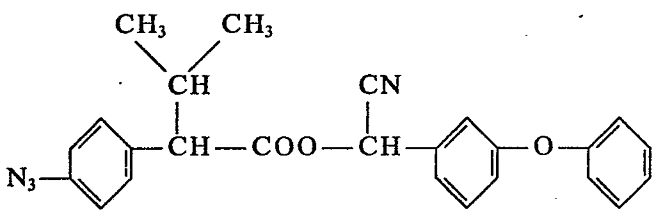

5. Verfahren zur Herstellung von Verbindungen gemäß Anspruch 1, dadurch gekennzeichnet, daß man eine Verbindung der Formel

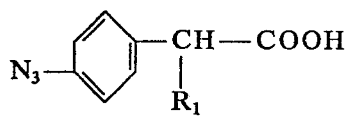

in Gegenwart eines säurebindenden Mittels mit einer Verbindung der Formel

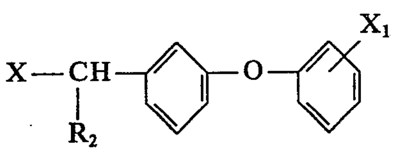

umsetzt, worin

$R_1$, $R_2$ und $X_1$ die im Anspruch 1 angegebene Bedeutung haben und
X für ein Halogenatom, insbesondere für Chlor oder Brom,

steht.

6. Ein Schädlingsbekämpfungsmittel, welches als aktive Komponente eine Verbindung gemäß Anspruch 1 und geeignete Träger- und/oder andere Zuschlagstoffe enthält.

7. Verwendung einer Verbindung gemäß Anspruch 1 zur Bekämpfung von verschiedenartigen tierischen und pflanzlichen Schädlingen.

8. Verwendung gemäß Anspruch 7 zur Bekämpfung von Insekten und Vertretern der Ordnung Akarina.

9. Eine Verbindung der Formel

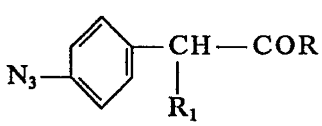

worin

$R_1$ Isopropyl oder Cyclopropyl und
R Hydroxy, Halogen oder $C_1-C_4$-Alkoxy

bedeuten.

**Claims**

1. A compound of the formula

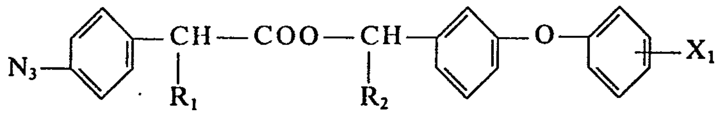

wherein

$R_1$ is isopropyl or cyclopropyl,
$R_2$ is hydrogen, cyano, ethynyl, prop-1-ynyl or $-CSNH_2$, and
$X_1$ is hydrogen, methyl, fluorine or chlorine.

2. A compound according to claim 1, wherein

$R_1$ is isopropyl or cyclopropyl,
$R_2$ is hydrogen, cyano, ethynyl, prop-1-ynyl or $-CSNH_2$, and
$X_1$ is hydrogen, p-fluorine or p-chlorine.

3. A compound according to claim 2, wherein

$R_1$ is isopropyl,
$R_2$ is cyano and
$X_1$ is hydrogen, p-fluorine or p-chlorine.

4. The compound according to claim 3 of the formula

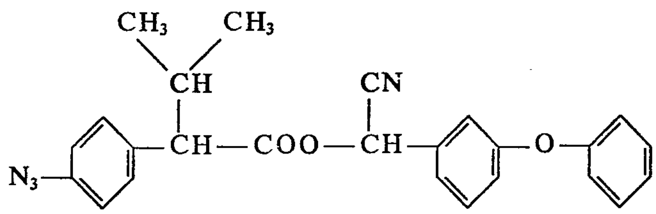

5. A process for the production of a compound according to claim 1, which process comprises reacting a compound of the formula

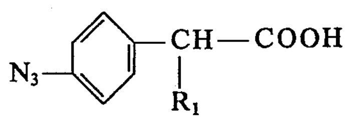

in the presence of an acid acceptor, with a compound of the formula

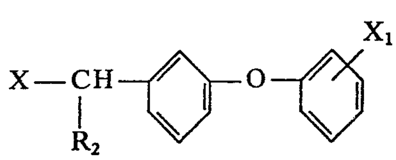

wherein

$R_1$, $R_2$ and $X_1$ are as defined in claim 1, and
X is a halogen atom, especially a chlorine or bromine atom.

6. A pesticidal composition which contains, as active component, a compound according to claim 1, together with suitable carriers and/or other adjuvants.

7. A method of controlling a variety of pests of animals and plants, which comprises the use of a compound according to claim 1.

8. A method according to claim 7, wherein the pests to be controlled are insects and representatives of the order Acarina.

9. A compound of the formula

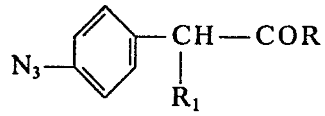

wherein

$R_1$ is isopropyl or cyclopropyl, and
R is hydroxy, halogen or $C_1-C_4$ alkoxy.

**Revendications**

1. Composé de formule

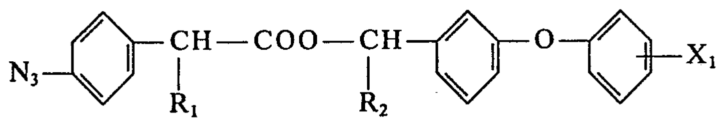

où

$R_1$ représente un isopropyle ou un cyclopropyle,
$R_2$ un hydrogène, un cyano, un éthynyle, un prop-1-ynyle ou $-CSNH_2$ et
$X_1$ représente un hydrogène, un méthyle, un fluor ou un chlore.

2. Composé selon la revendication 1, où

$R_1$ représente un isopropyle ou un cyclopropyle,
$R_2$ un hydrogène, un cyano, un éthynyle, un prop-1-ynyle ou $-CSNH_2$ et
$X_1$ représente un hydrogène, un p-fluor ou un p-chlore.

3. Composé selon la revendication 2, où

$R_1$ représente un isopropyle
$R_2$ un cyano et
$X_1$ un hydrogène, un p-fluor ou un p-chlore.

4. Composé selon la revendication 3 de formule

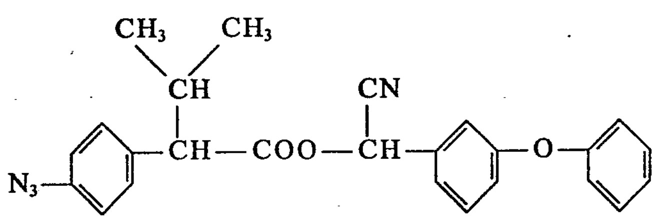

5. Procédé de préparation de composés selon la revendication 1, caractérisé en ce qu'on fait réagir un composé de formule

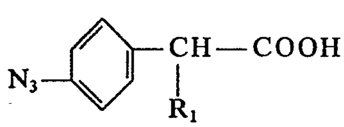

en présence d'un liant acide avec un composé de formule